# EUROPEAN PATENT APPLICATION

(11) **EP 3 495 973 A1**
(43) Date of publication of application: **12.06.2019**
(21) Application number: 17836214.1
(22) Date of filing: 08.06.2017
(51) Int. Cl.: G06F 19/00

(54) **METHOD AND DEVICE FOR PERFORMING TRANSFORMATION-BASED LEARNING ON MEDICAL IMAGE**

(30) Priority: 03.08.2016 CN 201610627265
(71) Applicant: Infervision, Beijing 100025 (CN)
(72) Inventor: CHEN, Kuan, Beijing 100025 (CN); ZHANG, Rongguo, Beijing 100025 (CN)
(74) Representative: Bell, Mark
(86) International application number: PCT/CN2017/087587
(87) International publication number: WO 2018/024031

(57) **Abstract**

The present application discloses a method and device for performing transformation-based learning on a medical image. The method comprises: reading raw data of a medical image, performing transformation processing on the data by analyzing a data attribute, and integrating the same into a data format capable of being received by a model to be analyzed; selecting a transformation mode by comparing parameters of the model to be analyzed and a trained model, so as to perform parameter transformation and apply transformation-based learning to training of the model to be analyzed for the medical image; and upon finishing model training, applying a parameter of a trained model to image category analysis. The method can increase the accuracy of a model obtained by performing deep learning on a small quantity of medical images. The invention further comprises a device for performing transformation-based learning on a medical image, comprising: a data processing module; a transformation-based learning module; and an application module.

## Description

### FIELD OF THE INVENTION

The present invention relates to the field of medical artificial intelligence and big data processing, in particular to a method and device for performing transfer learning on medical images.

### BACKGROUND OF THE INVENTION

With its strong rise, the new artificial intelligence technology having deep learning framework as its core has achieved considerable development and advancement in various fields. Breakthroughs are achieved in AlphaGo, automation vehicles, speech recognition and other technologies that people have been expecting for years in a very short time as well. In the foreseeable future, deep learning will also promote the applications of big data analysis and artificial intelligence in medical industry.

However, even with the above breakthroughs, currently deep learning technology still has relatively big problems in the training process:
i) Model training is costly. The best applied deep learning and big data analysis models are very large models. Time to train the model from the beginning is several weeks or even months, that is to say, model training consumes a lot of time and manpower. It is also not conducive to rapid iteration as well as research & development of the model by the modeler.
ii) Training data of some application scenarios are limited, the information contained in which are not enough to support the training of a large deep learning model, thus seriously limiting the range of application of deep learning model in such application scenarios.

Specific to the medical industry, the above two issues are embodied as follows:
1) The dimensions in medical field are more than that of the general application scenario. The diagnosis and treatment data of each patient are complicated. The deep learning and data analysis models are also larger and more complex than the general ones. The training costs are very high. Learning and training for deep learning and big data and machine learning models with traditional methods consume a lot of manpower and material resources, greatly reducing the economic feasibility of the application.
2) Although the overall data amount of medical field is large, data sources are distributed over hospitals, thus there are no unified database available. The amount of data (such as the number of cases) around a single application scenario is very limited. And the amount of data changes are insufficient to support training and computing large deep learning and big data models, therefore greatly limiting the scenarios in which deep learning and big data technologies are applied.

Both of the above-mentioned points greatly limit the development and continuous progress of the overall technology of deep learning in the medical industry.

In the training module of general deep learning and big data modeling, sample data are used to optimize and calculate the parameters in the model. Through analysis and calculation of the training module, deep learning and big data model parameters become more and more optimized, which ultimately leads to an optimized model that performs specific functions. Therefore, the optimization process through sample training is crucial in the modeling of big data, machine learning and deep learning.

Modeling in medical industry often requires complex and large deep learning and big data models to learn and train data because of complex scenes. However, the number of samples is often limited in different application scenarios. This brings a lot of pressure to the parameter optimization procedure in the deep learning and big data modeling training module. Usually, the amount of data we have is not enough to support the sample needed by the whole model to complete the optimization. In most cases the model is not effective enough after computation, and optimal points are not found in the optimization process.

### SUMMARY OF THE INVENTION

In order to overcome the deficiencies of the prior art, the present invention provides a method and device for performing transfer learning on medical images, which effectively solve the above optimization problems, and improve the practical effect of deep learning in the field of medical images.

A method for performing transfer learning on medical images of the present invention comprises the steps of:
S1: reading raw data information of medical images, transforming the data by analyzing a data attribute, and adjusting the data into a data format that is received by the model to be analyzed;
S2: selecting a transformation mode by comparing parameters of the model to be analyzed with a model already trained, performing parameter transformation using the selected transformation mode, and applying the transfer learning to the training of the model to be analyzed for medical images; and
S3: applying the parameters of the model already trained to the analysis for the category of images when the model training ends;

Wherein step S2, said parameters include parameters of the model to be analyzed and the model already trained; said transformation modes include a complete transformation mode, a partial transformation mode, a hybrid transformation mode, a parameter transform mode, a timing import mode and a customized import mode.

Preferably, the parameter transformation in step S2 comprises the steps of:
S21: reading parameters of the model already trained, and constructing a parameter reading interface according to the format of the model already trained;
S22: establish a pre-processing function for the group of parameters of the model already trained, including operations such as intercepting, segmenting, mathematically transforming, mixing, changing order of the data, and the like;
S23: importing a group of parameters of the model already trained after the completion of pre-processing into the model to be analyzed, and constructing a loading interface according to an input and output format of the model to be analyzed; and
S24: intervening in the analysis process of the model to be analyzed, and adjusting the parameter transformation according to the corresponding analysis in the training process of the model to be analyzed and the function parameters of the training module.

Preferably, the complete transformation mode comprises that: if the overall structures of the model to be analyzed and the model already trained are identical, having identical numbers of parameters and identical parameter structures, the parameters of the model already trained are directly as a whole transferred to the model to be analyzed to start training.

Preferably, the partial transformation mode comprises that: if the overall structures of the model to be analyzed and the model already trained are different, or if it is not desired to transfer all the parameters of the model already trained into the model to be analyzed, then some of the parameters of the model already trained are transferred into the model to be analyzed; or if it is not desired that all the parameters of the model to be analyzed come from the model already trained, then some of the parameters in the model to be analyzed are transferred from the model already trained, while the remaining parameters are generated and adjusted in other modes.

Preferably, the hybrid transformation mode comprises that: the parameters of the plurality of models already trained are combined in a specific manner simultaneously and transferred into the model to be analyzed.

Preferably, the parameter transform mode comprises that: during the process of transforming parameters of the model already trained into parameters of the model to be analyzed, the parameters of the model already trained undergone a specific mathematical transformation, and then be imported into the model to be analyzed.

Preferably, the timing import mode comprises that: during the process of transforming parameters of the model already trained into parameters of the model to be analyzed, the transformation is gradually performed during the training process according to the requirements, instead of being completed in one time.

Preferably, the customized import mode includes: during the process of transforming parameters of the model already trained into parameters of the model to be analyzed, the basic procedure, structure, objective, method and the like of the process of transforming parameters of the model already trained into parameters of the model to be analyzed are customized, so that the process of parameter import is more suitable for actual application scenarios.

The invention also relates to a device for performing transfer learning on medical images, comprising: a data processing module that reads raw data information of medical images, transforms the data by analyzing a data attribute, and adjusts the data into a data format that is received by the model to be analyzed; a transfer learning module that selects a transformation mode by comparing parameters of the model to be analyzed with the model already trained, performs parameter transformation using the selected transformation mode, and applies the transfer learning to the training of the model to be analyzed for medical images; an application module that applies the parameters of the model already trained to the analysis for the category of images when the model training ends; wherein, said parameters include parameters of the model to be analyzed and the model already trained; said transformation modes of the transfer learning module include a complete transformation mode, a partial transformation mode, a hybrid transformation mode, a parameter transform mode, a timing import mode and a customized import mode.

Preferably, the transfer learning module comprises that: a parameter acquisition sub-module that reads parameters of the model already trained, and constructs a parameter reading interface according to the format of the model already trained; a parameter pre-processing sub-module that performs pre-processing to the group of parameters of the model already trained, including operations such as intercepting, segmenting, mathematically transforming, mixing, changing order of the data, and the like; a parameter import sub-module that imports a group of parameters of the model already trained after the completion of pre-processing into the model to be analyzed, and constructs a loading interface according to an input and output format of the model to be analyzed; a model intervention sub-module that intervenes in the analysis process of the model to be analyzed, and adjusts the parameter transformation according to the corresponding analysis in the training process of the model to be analyzed and the function parameters of the training module.

The beneficial effects brought by the technical solution provided by the present invention are embodied in: before the deep learning model or the big data model begins to perform learning and optimization on the training data, parameters of a model trained in other application scenarios are loaded into the existing model, then the normal optimization operation of the model is started. The method and device provided by the invention help the model to start optimization at an ideal starting point in the parameter space that facilitates the model to find a better optimization point in a shorter time with less training samples, thus greatly lowers the cost and conditions of model training, allowing the model in more application scenarios to be applied. For example, we use the present invention to transfer the data model for analyzing femoral head to analyze and calculate thoracic pulmonary diseases for further improvement and analysis. For example, without using parameter transfer, when only pulmonary disease images are used for training, the accuracy of the model only reaches 65%. On the contrary, after transferring the parameters of the data model for analyzing femoral head to the pulmonary disease model, the accuracy of the training model reaches more than 85%, thus reducing the difficulty and cost of modeling and analysis of pulmonary diseases.

### DESCRIPTION OF THE DRAWINGS

In order to explain the technical solution in the embodiments of the present disclosure more clearly, the drawings that need to be used in the description of the embodiments will be briefly introduced below. Obviously, the drawings in the following description are only some embodiments of the present disclosure, and other drawings are obtained from these drawings without any creative work for those of ordinary skill in the art.
Figure 1 is a schematic diagram of a method for performing transfer learning on medical images according to an embodiment of the present invention;
Figure 2 is a flow chart of a method for performing transfer learning on medical images according to an embodiment of the present invention;
Figure 3 is a flow chart of the parameter transformation steps of the method in Figure 2;
Figure 4 is a block diagram of a device for performing transfer learning on medical images according to an embodiment of the present invention; and Figure 5 is a block diagram of the transfer learning module in Figure 3.

### DETAILED DESCRIPTION OF THE INVENTION

In order to make the object, technical solution and advantages of the present disclosure clearer, embodiments of the present disclosure will be described below in further detail with reference to the appended drawings.

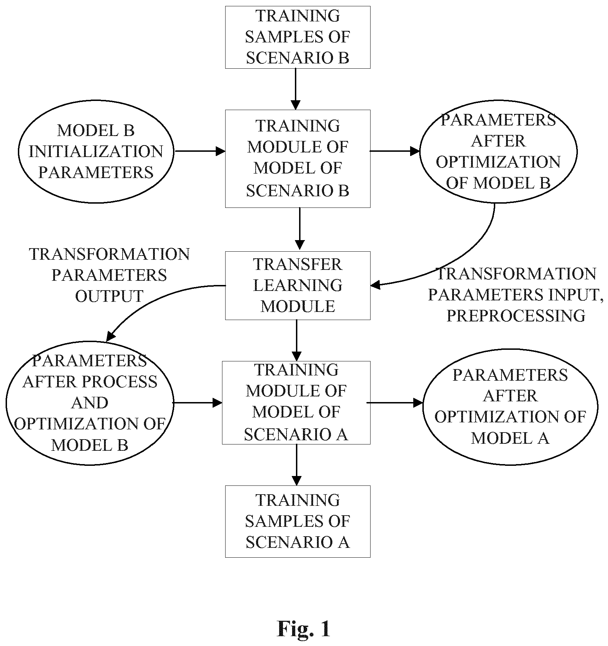

The present invention provides a method for performing transfer learning on medical images, as shown in Figure 1, which is a schematic diagram of a method of transfer learning according to an embodiment of the present invention.

Scenario A is a desired medical scenario that is learned and analyzed by deep learning, machine learning, or big data analysis. First, the training sample data related to scenario A are collected, and a training sample database is established. The model training module for scenario A is built on the basis of the training sample database, and the mathematical model used in the training module includes the basic framework (model layer) of the scenario A model and the parameters (parameter layer) of the framework. During the training process, the basic framework in the training module remains unchanged, but the parameters included in the basic framework are continuously updated, self-learned and optimized gradually round and round with the operation procedure, so that the model gradually possesses the expected analysis and intelligent effects.

When the model is very complex and the training samples are limited, the optimization process of scenario A is very difficult. In many cases, the model does not achieve the desired effect. The modeling and analysis process of scenario A therefore easily fail. Nonetheless the parameter transfer learning method effectively solves this problem. Scenario B is seen as another application scenario that has sufficient data and deep learning or big data model has very good effects.

Therefore, the parameter transformation function required for the transfer learning is completed by performing parameter comparison between model A (to be analyzed) and model B (already trained). Using the parameter transfer learning method, the trained parameters in scenario B are obtained, processed and preprocessed, and transferred to the basic model framework of model A.

Therefore, model A establishes its parameter optimization procedure on the transformed parameters. It is unnecessary for the training module of model A to optimize the parameters from the beginning. On the contrary, it suffices to just optimize the parameters of model B. The parameters with strong feature mining ability in model B are effectively transformed into the model of application scenario A by the model parameter transfer learning method.

After the completion of parameter transformation, medical images are trained in the deep learning model. After the training, the parameters of model B are applied to the analysis of relevant medical images. This step includes applying the transfer learning to the deep learning model training of the particular medical image. When the model training ends, the parameters of model B are applied to the analysis of categories of images.

For example, model A intends to train the deep learning model to analyze and mine the characteristics of the pulmonary nodules over X-ray, which assist diagnosis, but the model obtained by the analysis is less effective due to the limited data of pulmonary nodules is obtained. On the other hand, it is possible to have a large amount of X-ray data of pulmonary effusion and a deep learning model B for pulmonary effusion is trained successfully. Therefore, the parameters of the X-ray model of pulmonary effusion obtained by training model B are input to model A for pulmonary nodules to start learning. The training of model A greatly reduces the threshold of data volume because of the transformation of model B, thus the training effects are greatly improved.

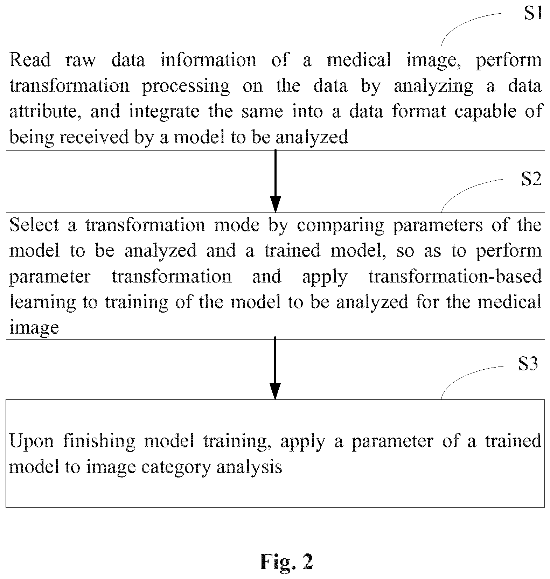

As shown in Figure 2, a method for transfer learning on medical images of the present invention includes the steps of:
S1: reading raw data information of medical images, transforming the data by analyzing a data attribute, and adjusting the data into a data format that is received by model A;
S2: selecting a transformation mode by comparing parameters of model A and model B, performing parameter transformation using the selected transformation mode, and applying the transfer learning to the training of model A of medical images;
S3: applying parameters of model B to the analysis for the category of images when the model training ends.

Wherein step S2, the said transformation modes include a complete transformation mode, a partial transformation mode, a hybrid transformation mode, a parameter transform mode, a timing import mode and a customized import mode.

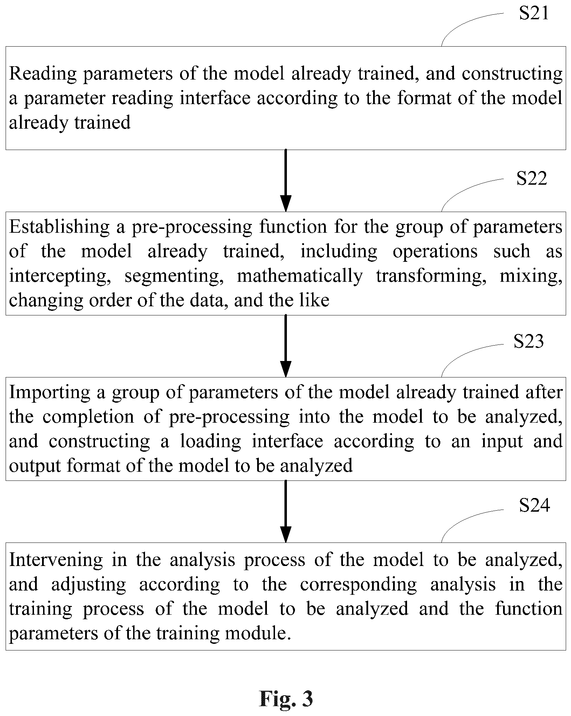

As shown in Figure 3, the parameter transformation in step S2 includes the steps of:
S21: reading the parameters of model B, and constructing a parameter reading interface according to the format of model B;
S22: establish a pre-processing function for the group of parameters of model B, including operations such as intercepting, segmenting, mathematically transforming, mixing, changing order of the data, and the like;
S23: importing a group of parameters of model B after the completion of pre-processing into model A, and constructing a loading interface according to an input and output format of model A;
S24: intervening in the analysis process of model A, and adjusting the parameter transformation according to the corresponding analysis in the training process of model A and the function parameters of the training module.

During the process of model A training, the training process of model A also is intervened to adjust a series of control parameters during the training process of model A. For example, the parameter transformation module selectively adjusts the learning speed of various parameters of model A, lowers the learning speed of lower-level parameters, and assigns higher learning speed to higher-level model parameters, so that various parameters have different learning and adjustment speeds.

The transformation modes of parameters of model B into parameters of model A include a complete transformation mode, a partial transformation mode, a hybrid transformation mode, a parameter transform mode, a timing import mode and a customized import mode. The complete transformation mode is that if the overall structures of model A and model B are identical, having identical numbers of parameters and identical parameter structures, the parameters of model B are directly as a whole transferred to model A to start training; the partial transformation mode is that if the overall structures of model A and model B are different, or if it is not desired to transfer all the parameters of model B into model A, the parameter transformation module still transfers some of the parameters of model B into model A; or if it is not desired that all the parameters of model A come from model B, then some of the parameters in model A are transferred from model B, while the remaining parameters are generated and adjusted in other modes; the hybrid transformation mode is that the parameters of the plurality of models (model B, model C, etc.) already trained are combined in a specific manner simultaneously and transferred into model A; the parameter transform mode is that during the process of the parameter transformation module transforming parameters of model B into parameters of model A, the parameters of model B undergo a specific mathematical transformation, and then be imported into model A; the timing import mode is that during the process of the parameter transformation module transforming parameters of model B into parameters of model A, the transformation is gradually performed during the training process according to the requirements, instead of being completed in one time; the customized import mode is that during the process of the parameter transformation module transforming parameters of model B into parameters of model A, the basic procedure, structure, objective, method and the like of the process of transforming parameters of model B into parameters of model A are customized, so that the process of parameter import is more suitable for actual application scenarios.

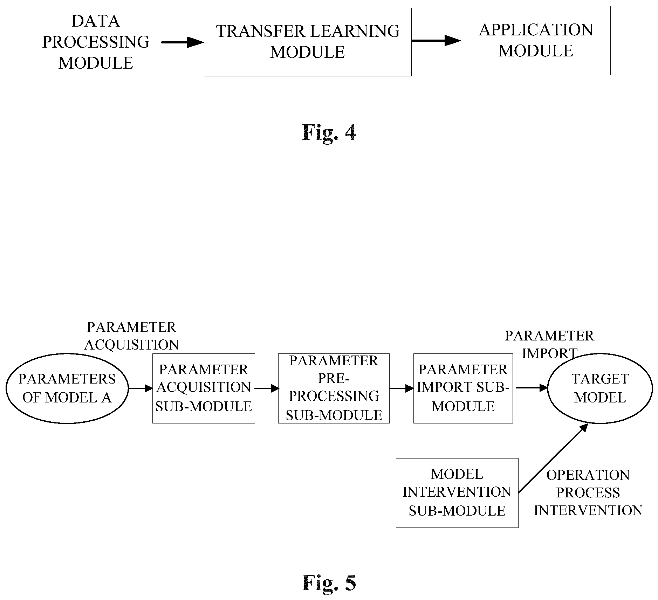

As shown in Figure 4, the present invention also relates to a device for performing transfer learning on medical images, the device comprising:
a data processing module that reads raw data information of medical images, transforms the data by analyzing a data attribute, and adjusts the data into a data format that is received by model A;
a transfer learning module that selects a transformation mode by comparing parameters of model A and model B, performs parameter transformation using the selected transformation mode, and applies the transfer learning to the training of model A of medical images;
an application module that applies the parameters of model B to the analysis for the category of images when the model training ends.

As shown in Figure 5, it illustrates a block diagram of the transfer learning module of Figure 4. The transfer learning module includes:
1) a parameter acquisition sub-module that reads parameters of model B, and constructs a parameter reading interface according to the format of model B;
2) a parameter pre-processing sub-module that performs pre-processing to the group of parameters of model B, including operations such as intercepting, segmenting, mathematically transforming, mixing, changing order of the data, and the like;
3) a parameter import sub-module that imports a group of parameters of model B after the completion of pre-processing into model A, and constructs a loading interface according to an input and output format of model A; and
4) a model intervention sub-module that intervenes in the analysis process of model A, and adjusts the parameter transformation according to the corresponding analysis in the training process of model A and the function parameters of the training module.

The following is given by way of example to illustrate.

First Embodiment: perform transfer learning on the parameters of an intelligent analysis chest X-ray model so that the parameters apply to an intelligent analysis chest CT model.

It is assumed that a large amount of data has been learned and analyzed to have a dedicated chest X-ray model for analysis. Assuming that the data image dimension of each X-ray image is (1, 4096, 4096), the dimension list of the model parameter matrix used is: [32, 3, 3, 3], [64, 32, 3, 3], [128, 64, 3, 3], [2, 4096] .

At this time, it is desired to analyze the chest CT model. Assuming that the image dimension of each slice of chest CT image is (300, 256, 256), and the model parameter list used is: [32, 300, 3, 3], [64, 32, 3, 3], [128, 64, 3, 3], [1000, 4096] .

The chest X-ray model has sufficient data amount and sufficient training, thus the model is very effective. On the contrary, the target training chest CT model has insufficient data amount and the model is huge and complex, therefore the training effect is far from ideal. Therefore, the device for transfer learning of parameters of models proposed by the present invention is used, wherein:
a data processing module that processes chest CT data into a format that is input into the chest CT model;
a transfer learning module that transforms the parameters of the chest X-ray model to train the chest CT model, which comprises that:
   a parameter acquisition sub-module that obtains all parameter matrices from the parameters of the chest X-ray model and input them into the parameter acquisition sub-module.
   a parameter pre-processing sub-module which pre-processes the first layer parameter using a unique transformation method because the parameter dimension of the chest X-ray model is different from the parameter dimension of the chest CT model, and this transformation makes use of the method that repeats the first layer parameter of the chest X-ray model 100 times on the second axis, with the last layer directly removed without any pre-processing as it is not loaded. The rest of the parameters are not pre-processed, and the transformation is directly performed, that is, the dimensions of the parameter matrices are transformed into [32, 3, 3, 3]→ [32, 300, 3, 3], [64, 32, 3, 3] → [64, 32, 3, 3], [128, 64, 3, 3] → [128, 64, 3, 3].
   a parameter import sub-module that loads the parameters except the last layer of parameters of the chest X-ray model into the chest CT model and begins the training of analysis, thus the last layer of parameters in the chest CT model will not be transformed.
   a model intervention sub-module that intervenes the model training process after the chest CT model starts training and learning, performs slower update and learning on the transformed first three layers of parameters, and assigns higher learning and update speeds to the last layer of parameters which are not transformed.
an application module which is entered after the chest CT model training is completed.

Second Embodiment: perform transfer learning on the parameters of an intelligent analysis femoral head X-ray model so that the parameters apply to an intelligent analysis cardiopulmonary X-ray model.

It is assumed that a large amount of data has been learned and analyzed with a dedicated femoral head model for analysis. Assuming that the data image dimension of each X-ray image is (1, 2000, 2000), the dimension list of the model parameter matrix used is: [32, 3, 3, 3], [64, 32, 3, 3], [128, 64, 3, 3], [2, 1024].

At this time, it is desired to analyze the cardiopulmonary X-ray model. Assuming that the image dimension of each slice of cardiopulmonary X-ray image is (1, 2000, 2000), and the model parameter list used is: [32, 3, 3, 3], [64, 32, 3, 3], [128, 64, 3, 3], [2, 1024].

The femoral head X-ray model has sufficient data amount and sufficient training, thus the model is very effective. On the contrary, the target training cardiopulmonary X-ray model has insufficient data amount therefore the training effect is far from ideal. Therefore, the device for transfer learning of parameters of models proposed by the present invention is used, wherein:
a data processing module that processes cardiopulmonary X-ray data into a format that is input into the cardiopulmonary X-ray model;
a transfer learning module that transforms the parameters of the femoral head X-ray model to train the cardiopulmonary X-ray model, which comprises that:
   a parameter acquisition sub-module that obtains all parameter matrices from the parameters of the femoral head X-ray model and input them into the parameter acquisition sub-module;
   a parameter pre-processing sub-module, wherein the transformation adopts the parameter complete transformation mode because the parameter dimensions of the femoral head X-ray model are identical to the parameter dimensions of the cardiopulmonary X-ray model.
   a parameter import sub-module which loads the parameters of parameters of the femoral head X-ray model except the last layer into the cardiopulmonary X-ray model and begins the training of analysis , thus the last layer of parameters in the cardiopulmonary X-ray model will not be transformed.
   a model intervention sub-module that intervenes the model training process after the cardiopulmonary X-ray model starts training and learning, performs slower update and learning on the transformed first three layers of parameters, and assigns higher learning and updates speeds to the last layer of parameters which are not transformed.
an application module which is entered after the cardiopulmonary X-ray model training is completed.

Third Embodiment: perform transfer learning on the parameters of an intelligent analysis pulmonary CT model so that the parameters apply to an intelligent analysis brain MRI model.

It is assumed that a large amount of data has been learned and analyzed with a dedicated pulmonary CT model for analysis. Assuming that the data image dimension of input into the CT model is (1,100, 512, 512), the dimension list of the model parameter matrix used is: [32, 100, 3, 3], [64, 32, 3, 3], [128, 64, 3, 3], [1, 1024].

At this time, it is desired to analyze the brain MRI model. Assuming that the image dimension of each slice of brain MRI image is (1,100, 256, 256), and the model parameter list used is: [32, 100, 3, 3], [64, 32 , 3, 3], [128, 64, 3, 3], [256, 128, 3, 3], [1, 1024].

The pulmonary CT model has sufficient data amount and sufficient training, thus the model is very effective. On the contrary, the target training brain MRI model has insufficient data amount, therefore the training effect is far from ideal. Therefore, the device for transfer learning of parameters of models proposed by the present invention is used, wherein:
a data processing module that processes brain MRI data into a format that is input into the brain MRI model;
a transfer learning module that transforms the parameters of the pulmonary CT model to train the brain MRI model, which comprises that:
   a parameter acquisition sub-module that obtains all parameter matrices from the parameters of the pulmonary CT model and input them into the parameter acquisition sub-module;
   a parameter pre-processing sub-module, wherein the transformation uses the parameter partial transformation mode because that although the parameter dimensions of the pulmonary CT model are identical to the parameter dimensions of the brain MRI model, the number of parameters are different.
   a parameter import sub-module which loads the parameters of parameters of the pulmonary CT model except the last layer into the brain MRI model, while the extra set of parameters [256, 128, 3, 3] of the brain MRI model adopts an initialization of random numbers following Gaussian distribution, then the training of analysis begins.
   a model intervention sub-module that intervenes the model training process after the brain MRI model starts training and learning, performs slower update and learning on the transformed first three layers of parameters, and assigns higher learning and updates speeds to the last layer of parameters which are not transformed.
an application module which is entered after the brain MRI model training is completed.

Specific embodiments of the present disclosure have been described above in detail, but it will be understood that modifications are made thereto without departing from the spirit of the present disclosure. The claims of the present disclosure are intended to cover these modifications so as to ensure that they fall within the true scope and spirit of the present disclosure.

## Claims

1. A method for performing transfer learning on medical images, **characterized by** comprising the steps of:
S1: reading raw data information of medical images, transforming the data by analyzing a data attribute, and adjusting the data into a data format that is received by the model to be analyzed;
S2: selecting a transformation mode by comparing parameters of the model to be analyzed with a model already trained, performing parameter transformation using the selected transformation mode, and applying the transfer learning to the training of the model to be analyzed for medical images; and,
S3:applying the parameters of the model already trained to the analysis for the category of images when the model training ends;
wherein at step S2, said parameters include parameters of the model to be analyzed and the model already trained; said transformation modes include a complete transformation mode, a partial transformation mode, a hybrid transformation mode, a parameter transform mode, a timing import mode and a customized import mode.

2. The method for performing transfer learning on medical images according to claim 1, **characterized in** the parameter transformation in step S2 comprises the steps of:
S21: reading parameters of the model already trained, and constructing a parameter reading interface according to the format of the model already trained;
S22: establish a pre-processing function for the group of parameters of the model already trained, including operations such as intercepting, segmenting, mathematically transforming, mixing, changing the order of the data, and the like;
S23: importing a group of parameters of the model already trained after the completion of pre-processing into the model to be analyzed, and constructing a loading interface according to an input and output format of the model to be analyzed; and
S24: intervening in the analysis process of the model to be analyzed, and adjusting the parameter transformation according to the corresponding analysis in the training process of the model to be analyzed and the function parameters of the training module.

3. The method for performing transfer learning on medical images according to claim 1, **characterized in** the complete transformation mode comprises that:
if the overall structures of the model to be analyzed and the model already trained are identical, having identical numbers of parameters and identical parameter structures, the parameters of the model already trained are directly as a whole transferred to the model to be analyzed to start training.

4. The method for performing transfer learning on medical images according to claim 1, **characterized in** the partial transformation mode comprises that:
if the overall structures of the model to be analyzed and the model already trained are different, or if it is not desired to transfer all the parameters of the model already trained into the model to be analyzed, then some of the parameters of the model already trained are transferred into the model to be analyzed; or if it is not desired that all the parameters of the model to be analyzed come from the model already trained, then some of the parameters in the model to be analyzed are transferred from the model already trained, while the remaining parameters are generated and adjusted in other modes.

5. The method for performing transfer learning on medical images according to claim 1, **characterized in** the hybrid transformation mode comprises that:
the parameters of the plurality of models already trained are combined in a specific manner simultaneously and transferred into the model to be analyzed.

6. The method for performing transfer learning on medical images according to claim 1, **characterized in** the parameter transform mode comprises that:
during the process of transforming parameters of the model already trained into parameters of the model to be analyzed, the parameters of the model already trained undergo a specific mathematical transformation, and are then imported into the model to be analyzed.

7. The method for performing transfer learning on medical images according to claim 1, **characterized in** the timing import mode comprises that:
during the process of transforming parameters of the model already trained into parameters of the model to be analyzed, the transformation is gradually performed during the training process according to the requirements, instead of being completed in one time.

8. The method for performing transfer learning on medical images according to claim 1, **characterized in** the customized import mode comprises that:
during the process of transforming parameters of the model already trained into parameters of the model to be analyzed, the basic procedure, structure, objective, method and the like of transforming parameters of the model already trained into parameters of the model to be analyzed are customized, so that the process of parameter import is more suitable for actual application scenarios.

9. A device for performing transfer learning on medical images, **characterized in** comprising:
a data processing module that reads raw data information of medical images, transforms the data by analyzing a data attribute, and adjusts the data into a data format that is received by the model to be analyzed;
a transfer learning module that selects a transformation mode by comparing parameters of the model to be analyzed with the model already trained, performs parameter transformation using the selected transformation mode, and applies the transfer learning to the training of the model to be analyzed for medical images;
an application module that applies the parameters of the model already trained to the analysis for the category of images when the model training ends;
wherein, said parameters include parameters of the model to be analyzed and the model already trained; said transformation modes of the transfer learning module include a complete transformation mode, a partial transformation mode, a hybrid transformation mode, a parameter transform mode, a timing import mode and a customized import mode.

10. The device for performing transfer learning on medical images according to claim 9, **characterized in** the transfer learning module comprises:
a parameter acquisition sub-module that reads parameters of the model already trained, and constructs a parameter reading interface according to the format of the model already trained;
a parameter pre-processing sub-module that performs pre-processing of the group of parameters of the model already trained, said pre-processing including operations such as intercepting, segmenting, mathematically transforming, mixing, changing order of the data, and the like;
a parameter import sub-module that imports a group of parameters of the model already trained after the completion of pre-processing into the model to be analyzed, and constructs a loading interface according to an input and output format of the model to be analyzed; and,
a model intervention sub-module that intervenes in the analysis process of the model to be analyzed, and adjusts the parameter transformation according to the corresponding analysis in the training process of the model to be analyzed and the function parameters of the training module.
